Europäisches Patentamt

European Patent Office

Office européen des brevets

(19)

(11) Publication number: **0 230 983 B1**

(12) **EUROPEAN PATENT SPECIFICATION**

(45) Date of publication of patent specification: **24.07.91**

(51) Int. Cl.⁵: **C07C 2/30, B01J 31/14, B01J 31/26**

(21) Application number: **87100821.5**

(22) Date of filing: **22.01.87**

(54) **Ethylene dimerization and polymerization.**

(30) Priority: **27.01.86 US 822453**

(43) Date of publication of application:
**05.08.87 Bulletin 87/32**

(45) Publication of the grant of the patent:
**24.07.91 Bulletin 91/30**

(84) Designated Contracting States:
**AT BE CH DE ES FR GB IT LI LU NL SE**

(56) References cited:
**DE-A- 1 803 434**
**US-A- 2 943 125**

(73) Proprietor: **PHILLIPS PETROLEUM COMPANY**
**5th and Keeler**
**Bartlesville Oklahoma 74004(US)**

(72) Inventor: **McDaniel, Max Paul**
**1610 SE Melmart Drive**
**Bartlesville, OK 74006(US)**
Inventor: **Smith, Paul David**
**4927 Feagan**
**Houston, TX 77007(US)**
Inventor: **Klendworth, Douglas Dean**
**3211 Wayside Drive**
**Bartlesville, OK 74006(US)**

(74) Representative: **Dost, Wolfgang, Dr. rer. nat.**
**Dipl.-Chem. et al**
**Patent- u. Rechtsanwälte Bardehle, Pagen-**
**berg, Dost, Altenburg Frohwitter & Partner**
**Galileiplatz 1**
**W-8000 München 80(DE)**

**Description**

The present invention relates to the conversion of ethylene to dimers, oligomers and higher polymers. In one aspect the present invention relates to a process specifically useful for the oligomerization of ethylene predominately to α-olefins.

The catalysts generally employed for the oligomerization of ethylene are the result of the combination of Group VIII metal compounds and metal alkyl reducing agents. Examples are disclosed in U.S. Patent No. 4,032,590.

There have been disclosures of the use of soluble complexes of transition metal esters and trialkyl aluminums as catalysts for the dimerization and oligomerization of ethylene. See for example, U.S. Patent No. 2,943,125 "Journal Of Polymer Science, Vol. XXXIV, pp. 139-151 (1959) and DE-A-1 803434.

Such transition metal based dimerization catalysts have even been proposed for use with polymerization catalysts to result in the in-situ dimerization of ethylene so that ethylene copolymers could be made without the employment of a separate comonomer stream. See U.S. Patent No. 4,133,944 and "Journal Of Polymer Science, Vol. XXII, pp. 3027-3042 (1984). These liquid transition metal catalyst compositions have been found to have a rapid drop-off in activity. In addition they have been found to tend to poison polymerization catalysts, thus limiting their usefulness in the cojoint dimerization and polymerization of ethylene.

One object of the present invention is to provide a dimerization process using a solid transition metal based catalyst having high activity and high selectivity to α-olefins.

Another object of the present invention is to provide a dimerization process having a more steady kinetic profile than provided prior art soluble transition metal catalysts.

Still another object of the present invention is to provide a transition metal dimerization process which does not tend to poison polymerization catalysts as do the prior art soluble catalysts.

Yet another object of the present invention is to provide a transition metal dimerization process which produces less gumming and fouling of the dimerization reactor than do prior art soluble transition metal catalysts.

Summary of the Invention

Accordingly, the invention relates to a process for the production of ethylene dimer comprising subjecting ethylene to dimerization in the presence of a dimerization catalyst system comprising a catalyst including a non-halogenated titanium compound which contains three or more -OR groups bonded to the titanium atom wherein each R is individually selected from hydrocarbyl groups containing 1 to 10 carbon atoms and a cocatalyst comprising a trialkylaluminum reducing agent, which process is characterized by utilizing a catalyst system wherein said catalyst is bonded to an insoluble solid being a finely divided inorganic oxidic support.

Detailed Description

The dimerization in accordance with this invention uses an insoluble solid having at least on the surface thereof hydrocarbyl ortho ester groups bonded to titanium. The term hydrocarbyl ortho ester group is intended to denote groups of the formula -OR wherein R is a hydrocarbyl radical having from 1 to 10 carbon atoms.

Such materials which will remain solid during the dimerization can be formed by various means known in the art. One technique involves using a silica-titania cogel which has been treated with an alcohol, such as methanol to form bonds between the titanium and methoxy groups.

The currently preferred technique involves treating an inorganic porous solid particulate support with a hydrocarbyl ester of titanium.

The supports used in making the dimerization catalyst in that manner are porous finely divided inorganic oxidic materials. Examples of typical oxidic materials include silica, alumina, silica-alumina, zirconia, thoria, magnesium oxide, aluminum phosphate and phosphated alumina. More specific examples include Davison

(TM)

2

952 grade silica, aluminum phosphate, especially those having a phosphorus to aluminum ratio in the range of 0.6/1 to 0.9/1, and supports of the type disclosed in U.S. Patent No. 4,364,841. Preferably the support has a high surface area and a large pore volume. The optimum particle size for the supports can be determined by routine experimentation. Typically, such supports have particle sizes in the range of 200 to 0.1 $\mu$m. The internal porosity may be measured as the ratio between the pore volume and the weight of the support and can be determined by the BET-technique, described by S. Brunauer, P. Emmett, E. Teller in "Journal Of The American Chemical Society, 60, pp. 209-319 (1938). Especially suitable are those supports having an internal porosity of at least about 0.6 cm$^3$/g. The surface area of the supports can be determined according to the above-mentioned BET technique, with the further use of the standardized method as described in British Standards, BS 4358. Volume 1 (1969). Typically the surface area will be in the range of 100 to 600, more preferably 300 to 500 square meters per gram.

The support is generally calcined or otherwise treated with an oxygen-containing media such as air at an elevated temperature to dry and/or activate the support, as is common in the art, before the support is treated with the transition metal compound. As is known in the art activation temperatures vary depending upon the support selected. Aluminum phosphate supports are generally activated at a temperature in the range of 150 to 800° C, more commonly at a temperature in the range of 400 to 600° C. It has been noted that the higher activation temperatures sometimes result in a more active catalyst.

It is within the scope of the present invention to employ supports of the type described above which have been treated with organometallic reducing agents. Some of the preferred organometallic reducing agents are organomagnesium compounds of the formula MgR$_2$, wherein each R is independently selected from hydrocarbyl groups, most preferably alkyl groups, containing 1 to 30 carbon atoms; organoaluminum compounds of the formula AlR$_3$, wherein each R is selected from alkyl groups containing 1 to 12 carbon atoms; and mixtures thereof. Typical examples include triethyaluminum, tri-isobutyl aluminum, dibutyl magnesium, diethyl magnesium, dihexyl magnesium, dioctyl magnesium and organomagnesium-aluminum complexes of the general formula (MgR$_2$) m (AlR'$_3$)n, particularly those wherein the ratio of m/n is in the range of 2/1 to 10/1. Such organomagnesium aluminum complexes are disclosed in U.S. 4.004, 071.

The treatment of the support with organometallic reducing agents is generally carried out by contacting the activated support with a solution of the reducing agent. Typical solvents include straight or branched saturated aliphatic hydrocarbons, such as butanes, pentanes, hexanes, heptanes, or commonly available mixtures such as gasoline, kerosene or gas oil. Cyclic hydrocarbons, like cyclopentane, cyclohexane, methylcyclohexane, and aromatic hydrocarbons such as benzene or chlorobenzene are also examples. From a practical point of view it is preferable to employ aliphatic hydrocarbons which are liquid at normal room temperatures but which can be readily removed by distillation.

Preferably when supports are produced using the organometallic reducing agents, the solution of the reducing agent and the support is heated to insure optimum bonding of the reducing agent to the support, typically this would be at temperatures in the range of 80° C to 300° C, more generally 100° C to 200° C. The pressure at which the treatment is carried out and the atmosphere employed is not particularly critical, but generally it would be carried out at about atmospheric pressure and in a non-oxidizing environment. The duration of the heat treatment necessary for obtaining optimum bonding at a given temperature can be determined by routine experimentation. Typically, it is sufficient to merely reflux the solution on the support for 10 minutes to an hour.

After the reaction between the support and the reducing agent has been completed the liquid and the solid are preferably separated before the support is contacted with the transition metal compound. This can be done by decanting, for example. It is generally desirable also to wash the solid several times with a solvent to remove reducing agent that is not bound to the support.

The quantities of reducing agent employed are not particularly critical. Obviously there is no advantage to using more reducing agent than can be bonded to the support since the unbonded material is preferably removed by the solvent when the dry solid is recovered.

The titanium compounds employed in the present invention are non-halogenated compounds of titanium containing three or more OR" groups bonded to the titanium wherein each R" is individually selected from hydrocarbyl groups containing 1 to 10, more preferably 2 to 10 carbon atoms. Typically, it is preferred to employ compounds in which the R" groups are alkyl or alkaryl. Some specific examples of such compounds include butyl titanate polymer, Ti(OC$_2$H$_5$)$_4$, Ti(OC$_3$H$_7$)$_4$, Ti(OiC$_3$H$_7$)$_4$, Ti(OnC$_4$H$_9$)$_4$, and Ti-(OC$_6$H$_4$CH$_3$)$_4$. The most preferred titanium compounds are those that are soluble in n-heptane at temperatures in the range of 50 to 70° C.

The treatment of the support with the titanium compound is generally carried out by contacting the support with liquid containing the titanium compound. It is generally preferred to use solutions of the titanium compound.

Solvents employed can be of the same type as described above for use with reducing agents. The support is contacted with the titanium compound under conditions conducive to the bonding of titanium metal to the support. The temperature, pressure, and time employed for the contacting can vary over a wide range. Typically the temperature would be in the range of 50°C to 300°C more preferably in the range of 50°C to 100°C. As a matter of convenience the contacting would generally be conducted at about atmospheric pressure. The duration of contacting necessary for obtaining optimum bonding at a given temperature can be determined by routine experimentation. Typically, it is sufficient to merely reflux the solution over the support for 5 minutes to an hour.

After the reaction between the support and the titanium compound, the liquid and the solid are separated. This can be done by evaporating or decanting. Generally it is preferable to wash the solid several times with a solvent to remove soluble transition metal remaining on the support.

If the resulting titanium containing support has not been subjected to temperatures of at least about 80°C since the titanium compound was added it is generally desirable to heat it for some time at a temperature of at least 80°C to 200°C to ensure total activation of the catalyst before it is used in the dimerization.

The amount of titanium added to the support can vary over a wide range. Generally, however, it is desirable to obtain as much bound titanium as possible in the support, thus it is not unusual to produce catalysts containing 5 wt. % of titanium calculated on an elemental basis, more typically the catalyst will contain 2 to 4 weight percent of titanium.

When the catalyst is one which has been prepared using a support which has been treated with reducing agent as described above, it is generally desirable to prepare catalysts in which the molar ratio of titanium to the metal of the reducing agent, i.e., magnesium and/or aluminum, is at least about 1/1, more preferably greater than 1/1, most preferably 1 mole per reducing equivalent.

The solid dimerization catalyst is employed in conjunction with a cocatalyst comprising a trialkylaluminum reducing agent. The particularly preferred reducing agents are trialkyl aluminum compounds of the type mentioned earlier.

The amount of cocatalyst employed can range over a fairly wide range, however the amount of cocatalyst employed can affect the catalyst activity. The amount of cocatalyst necessary for optimum activity will vary, depending upon the particular cocatalyst and dimerization catalysts employed. Typically, however the molar ratio of the metal of the reducing agent cocatalyst to the transition metal of the solid dimerization catalyst will be in the range of 5/1 to 1/5, more preferably 2/1 to 1/1.

The reaction conditions for the dimerization can also vary over a wide range. The reaction is effected by merely contacting ethylene with the mixture of the catalyst and cocatalyst. The ethylene pressure typically would be in the range of 0.2 to 20 atmospheres, more preferably 1 to 10 atmospheres. It is within the scope of the invention to have hydrogen present during the reaction. In fact in many cases the employment of hydrogen results in increased activity. When employed the hydrogen pressure would generally be in the range of 0.1 to 0.6 MPa (1 to 6 atmospheres).

The temperature at which the reaction is begun can also vary over a wide range, typically in the range of 20°C to 150°C, though temperatures in the range of 30°C and 100°C would be more common, with temperatures in the range of 50 to 100°C being generally most preferred.

In an especially preferred embodiment of the present invention the solid dimerization catalyst is used in conjunction with an ethylene polymerization catalyst so that ethylene copolymers can be produced directly from ethylene.

The polymerization catalyst employed can be a solid transition metal polymerization catalyst. Many such catalysts are known in the art and are available commercially. Typically the catalysts are based on the transition metals titanium, zirconium, or vanadium. Some typical examples of such catalysts include the Stauffer-type TiCl$_3$•0.33AlCl$_3$ and titanium, magnesium-containing catalysts such as disclosed in U.S. patents 4,198,718; 4,069,169; 4,327,158; 4,325,837; 4,326,988; 4,400,303; 4,394,291; 4,391,736, 4,363,746 and 4,312,784. The cojoint dimerization/polymerization is conducted using the same types of reaction conditions normally employed with the polymerization catalysts, including the use of the cocatalysts generally used with those catalysts. Generally the preferred cocatalysts are trialkylaluminum compound in which the alkyl groups contain no more than about four carbon atoms. Since it has been noted that organoaluminum halides generally have an adverse effect on the activity of the dimerization catalyst it is generally preferable to use polymerization catalyst/cocatalyst combinations that are free of significant amounts of soluble organoaluminum halides. Examples of particularly useful cocatalysts include triethylaluminum and trimethylaluminum.

Since the amount of cocatalyst employed can affect the activity of the dimerization catalyst, the amount of cocatalyst employed with the polymerization catalyst will vary depending upon the results desired.

Further, it is noted that as the ratio of the dimerization catalyst to polymerization catalyst increases the density of the polymer generally decreases which indicates that larger amounts of dimerization are occurring. Accordingly the ratio of the dimerization catalyst to the polymerization catalyst can be varied depending on the results desired.

A further understanding of the present invention and its advantages will be provided by a review of the following examples.

EXAMPLE I (Control)
Dimerization in liquid catalyst

A liquid catalyst was prepared by adding 3.0 ml of $Ti(OC_2H_5)_4$ to 100 ml of toluene and then adding 5.33 ml of a 15 weight percent solution of triethylaluminum. An emerald green solution resulted. To carry out the dimerization 16 ml of the emerald green solution was added to an autoclave along with 1.0 ml of the 15 weight percent triethyl aluminum solution and one liter of isobutane at 80° C. Ethylene was then supplied on demand at 0.38 MPa gauge (550 psig). Only a weak reaction of the ethylene was observed. After 20 minutes another 1 ml of the TEA solution was added and the rate soared to 578 gms/hr. Within 10 minutes later it had dropped to 348 gm/hr. Nine minutes later another ml of the TEA solution was added, and the rate picked up at 848 gm/hr but soon began dropping again and was 448 gm/hr by the time 1 hr had passed since the dimerization reaction had first begun. The reactor when opened was found to contain a scum of polymer.

A second experiment was carried out in the same manner except that this time even more TEA was added to explore further the effect of the TEA. The results can be summarized as follows:

| TEA/Ti Ratio | 0.5 | 1.0 | 1.6 | 2.0 | 2.9 |
|---|---|---|---|---|---|
| Initial Consumption | 0 | 578 | 848 | 1008 | 0 |

From this it was concluded that if the amount of TEA was too high it had an adverse effect on the activity. It was also noted that the activity always dropped quickly after the addition of the TEA, regardless of the TEA/Ti ratio. The activity of the total runs in terms of grams of ethylene consumed per gram of titanium for the two one hour reactions were calculated to be 2975 g/g Ti-hr and 2025 g/g Ti-hr, respectively. It should be noted however, that since the activity drops quickly with time these catalysts had very little activity shortly after each TEA addition. Accordingly, these values are unrealistically high if one is concerned with a steady reaction rate.

EXAMPLE II (Control)
Dimerization in Liquid Catalyst

In this run 2.0 ml of $Ti(OEt)_4$ was added to 24.6 g of toluene and then 11.5 mls of magala (a solution of 7 moles ethyl-butyl magnesium and 1 mole trialkyl aluminum) was added with slow stirring. This was a Mg/Ti molar ratio of 0.5/1. The solution turned blue-green. After setting overnight a fine blue-green precipitate formed. Before that, however, 16 ml of the blue-green solution was added to the reactor as in Example 1 with 1 ml of the TEA solution. Upon supplying ethylene there was an active consumption of ethylene but within about 10 minutes the activity was only about half that of the initial activity. Another ml of TEA was added, but only a minor improvement in activity resulted. After 48 minutes, the reaction was shut down, since the activity had dropped to only 19% of its initial value. The activity on an hourly basis for this run was calculated as 1265 g/g Ti-hr.

A duplicate of the above experiment was conducted. It gave an activity of 1900 g/g Ti-hr. Again it was noted that there was a severe loss in activity within about 10 minutes after the TEA was added.

EXAMPLE III
Solid Catalyst A Preparation

An aluminophosphate having a phosphorus/aluminum ratio of about 0.9 was calcined in air for 3 hours at 600° C. Then 7.3 gm of the calcined support was slurried in about 30 ml of heptane, to which 16.8 ml of magala solution was added. This was calculated to be an amount of magnesium equal to about 5% of the support weight. The slurry was refluxed for ten minutes, then the excess was drained off, and the solid rinsed in heptane twice to remove remaining soluble magnesium. The 7.7 ml of titanium tetraethoxide was added to the support. This was calculated as being about 2.5 moles of Ti per mole of Mg. This resulted in a blue-green solid and a blue solution. The slurry was refluxed five minutes, the liquid was then drained off and the solid was washed several times with isopentane. The solid was then dried under flowing nitrogen on a hot plate at 50-100°C.

EXAMPLE IV
Dimerization with Solid Catalyst A

Exactly 0.9111 gm of Catalyst A was introduced into a 2 liter autoclave along with 1.0 ml of a 15 weight percent solution of triethyl aluminum and one liter of isobutane 80°C. Ethylene was then supplied on demand at 0.38 MPa gauge (550 psig). Immediately a large heat of reaction was noticed a consumption of ethylene of at least 600 g/hr was observed. The activity was high and substantially constant throughout the reaction which was stopped 30 minutes later. Integration of the flow rate indicated that at least 225 mg of ethylene had been consumed. This gives an activity in terms of grams of ethylene consumed per gram of titanium per hour of 12,350! When the reactor door was opened, no polymer was found, only a liquid having a strong olefinic odor.

Another dimerization reaction was carried out with Catalyst A using a smaller Ti/TEA ratio. In this case 0.7701 gms of the solid catalyst was used with 2.0 ml of the TEA solution. Again a strong reaction was obtained which remained strong over the 30 minute reaction period. The activity was calculated as 12,500 grams of ethylene/gram Ti-hr. The reactor was found to contain about 6 gms of polymer. The solution in the reactor was analyzed by GLC and the results were as follows:

1-butene 96.5 mole %
2-butene trace
hexenes 2.9 mole %
heptenes 0.3 mole %
octenes 0.2 mole %

Thus the reaction was highly selective to the production of 1-butene.

Another dimerization reaction was conducted with Catalyst A, this time employing hydrogen. Exactly 0.7228 gm of A was added along with 1 mL of the TEA solution and 0.69 MPa (100 psi) of hydrogen. The ethylene supplied at 0.31 MPa (450 psi). This time an even stronger reaction was obtained, giving a steady flow rate of over 700 gm/hr. The integral indicated that 308 grams of ethylene had been consumed in 26 minutes, giving an activity of 24,700 gms of ethylene/gm Ti-hr. Only a trace of polymer was found.

The solution in the reactor was analyzed by GLC and the results were as follows:

1-butene 87.2 mole %
cis-2-butene 0.6 mole %
trans-2-butene 1.4 mole %
hexenes and higher 10.7 mole %

The hydrogen thus improved the activity of the catalyst without changing its selectivity to 1-butene very much.

EXAMPLE V

A solid was prepared in the same manner as described in Example III except that in the last step the isopentane wash liquid was merely evaporated at room temperature rather than having the solid heated at 50 to 100°C on a hot plate under flowing nitrogen. This gave a blue-purple solid which did not catalyze a reaction with ethylene.

EXAMPLE VI

The solid of Example V was heated in nitrogen at 100°C for a few minutes and it turned green. This green solid will be referred to as Catalyst B. In a dimerization run 0.7393 gm of Catalyst B gave a flow of over 400 gm of ethylene/hr. This calculated as an activity of 12,350 gms of ethylene/gm of Ti-hr. The activity was almost constant over the whole 30-minute reaction time.

EXAMPLE VII
Silica as Support

Solid catalysts were prepared using Davison 952 grade silica as the support. The support was calcined at 400° C in air.

In one case 8.0 gms of the calcined silica was slurried in a hydrocarbon and 8 mols of the magala solution was added. The slurry was refluxed, the excess liquid drained off and the solid rinsed with hydrocarbon. The solid was then contacted with 3.2 ml of titanium tetraethoxide at 80° C. The solid was then washed with hydrocarbon and dried over low heat in a nitrogen stream to give Catalyst C.

A Catalyst D was prepared in a similar manner using 9.3 gms of the calcined silica, 9.3 ml of the magala solution, and 1.5 ml of the titanium tetraethoxide.

Catalyst C and D were tested in a 2-liter reactor under particle form conditions using a TEA cocatalyst and 50 psig hydrogen. Catalyst C gave an activity of 14,750 gms of ethylene per gram of Ti-hr. Catalyst D gave an activity of 16,750 gms of ethylene per gram of Ti-hr. Only minor amounts of polymer were obtained. GLC of the non-polymeric products showed the catalysts to be highly selective to 1-butene. Greater than 98 mole percent of the liquid was 1-butene.

EXAMPLE VIII
Catalyst Prepared Without Reductant

Catalysts were prepared by contacting calcined Davidson 952 grade silica with Ti(OEt)$_4$ at 80° C, washing off the excess titanium and then drying over low heat in a nitrogen stream.

Catalyst E was prepared using 7.0 gm of the silica and 7 ml of Ti(OEt)$_4$.

Catalyst F was prepared using 5.4 gm of the silica and 5.4 gm of the Ti(OEt)$_4$.

When employed in contact with TEA on ethylene both catalysts gave high activities which dropped off much less rapidly with time than did the liquid catalysts. Both gave only minor amounts of polymer. Catalyst E gave an activity of 15,900 gms of ethylene/gm Ti-hr and Catalyst F 13,350 gm/Ti-hr.

EXAMPLE IX
Cojoint Dimerization/Polymerization With Liquid Catalyst

Tests were made to evaluate suggestions made in the literature about using the liquid catalysts of Example I in conjunction with a polymerization catalyst to cause the cojoint dimerization and polymerization of ethylene to give ethylene copolymers.

The polymerization catalyst employed was a commercial catalyst of the type sold by Catalyst Resources, Inc. under the trade name Lynx 705. That catalyst is one of the type disclosed in U.S. Patent No. 4,347,158.

The Lynx 705

(™)

catalyst when used in the amount of 0.0489 gm along with 1 ml of TEA gave 4622 gm of polymer in 26 minutes. There was no evidence of olefin production.

To carry out a cojoint polymerization/dimerization 0.0691 gm of Lynx 705 catalyst and 8 ml of the blue-green solution of Example II were added along with 2 ml of TEA to the reactor. Ethylene consumption started at 336 gm/hr but dropped quickly to 73 gm/hr in 20 minutes. Only a scum of polymer was found in the reactor.

In another run 0.1289 of the Lynx 705 catalyst and 1 ml of TEA were employed and 0.5 ml of Ti(OEt)$_4$ was employed in place of the blue-green solution. There was no evidence of either polymerization or dimerization. This shows that the soluble Ti(OEt)$_4$ tends to poison the polymerization catalyst. A similar observation is reported in "Journal Of Polymer Science", Polymer Chemistry Edition, Vol. 22, pp. 3034 and 3037.

EXAMPLE X
Cojoint Dimerization/Polymerization With Solid Catalyst

A solid dimerization catalyst was made by reacting 10.5 gm of Davison

⊕

952 grade silica calcined at 400°C with 10.5 ml of Ti(OEt)$_4$ in heptane slurry and then rinsing off the excess Ti with three heptane washes. This catalyst in the amount of 0.4521 gm, was charged to the reactor along with 0.0621 gm of Lynx 705 catalyst and 1 ml of TEA. A vigorous reaction started and grew rapidly until by 10 minutes the reaction had to be shut down because it could not be controlled, due to overheating. When the reactor was opened, 45 gm of polymer was recovered which smelled strongly of butene. Calculated productivity was 725 g/gm in 10 minutes which is close to that obtained in a control run in which 0.0357 gm of the Lynx 705 catalyst was used with 1 ml of TEA in the absence of dimerization catalyst.

Since it is known that copolymerization generally never occurs as easily as homopolymerization it thus follows that the solid dimerization catalyst did not give any evidence of poisoning the polymerization catalyst.

EXAMPLE XI
Solid Vanadium Dimerization Catalyst

A dimerization catalyst was prepared by dissolving 3.0 gms of VO(OiPr)$_3$ in 30 ml of anhydrous heptane. The solution was mixed with 11.40 grams of Davison 952 grade silica which had been calcined at 400°C. The solid and the liquid were separated and the solid dried as in the previous preps by heating on a hot plate under a stream of nitrogen.

In one experiment 2.0821 gms of the solid catalyst was added to the reactor with 1 ml of the TEA solution. In 30 minutes 39 grams of product was made, of which 36 grams were polymer. A strong odor of butene and hexene was detected in the reactor and on the polymeric product. The conditions were 100°C, 0.38 MPa (550 psi) ethylene and 1 liter of isobutane.

In another experiment the catalyst was used in combination with hydrogen. In this case 65 grams of product was produced in 30 minutes, of which 43 grams was polymer. Again a strong odor of butene and hexene was detected in the reactor and on the polymeric product.

**Claims**

1. A process for the production of ethylene dimer comprising subjecting ethylene to dimerization in the presence of a dimerization catalyst system comprising a catalyst including a non-halogenated titanium compound which contains three or more -OR groups bonded to the titanium atom wherein each R is individually selected from hydrocarbyl groups containing 1 to 10 carbon atoms and a cocatalyst comprising a trialkylaluminum reducing agent
   **characterized** by utilizing a catalyst system wherein said catalyst is bonded to an insoluble solid being a finely divided inorganic oxidic support.

2. The process of claim 1 characterized in that said support is selected from silica, alumina, silica-alumina, zirconia, thoria, magnesium oxide, aluminum phosphate and phosphated alumina.

3. The process of claim 2 wherein said support is aluminum phosphate or silica.

4. The process of any of the preceding claims, characterized in that said titanium compound is selected from Ti(OC$_2$H$_5$)$_4$, Ti(OC$_3$H$_7$)$_4$, Ti(OiC$_3$H$_7$)$_4$, Ti(OnC$_4$H$_9$)$_4$ and Ti(OC$_6$H$_4$CH$_3$)$_4$.

5. The process of any of the preceding claims characterized in that said transition metal compound is Ti-(OC$_2$H$_5$)$_4$.

6. The process of any of the preceding claims characterized in that said cocatalyst is triethylaluminum.

7. The process of any of the preceding claims characterized in that said support has been treated with organometallic reducing agent selected from compounds of the formula MgR$_2$ and AlR'$_3$, wherein each R is independently selected from hydrocarbyl groups containing 1 to 30 carbon atoms and each R' is independently selected from alkyl groups containing 1 to 12 carbon atoms.

8. The process of claim 7 wherein said support has been treated with ethyl butyl magnesium.

EP 0 230 983 B1

9. The process of any of the preceding claims characterized in that said dimerization is carried out in the further presence of a solid transition metal polymerization catalyst to obtain an ethylene copolymer.

10. The process of claim 9 wherein said solid transition metal polymerization catalyst contains titanium.

11. The process of claim 1 characterized in that said solid bonded catalyst is prepared by reacting a silica-titania cogel with methanol to form bonds between the titanium atoms and the methoxy groups.

12. The process of claim 1 characterized in that said solid bonded catalyst comprises butyl titanate polymer as transition metal compound.

13. The process of claim 1, characterized in that said catalyst has been prepared by treating said support with said titanium compound in a solvent at a temperature from 50 to 300 $^\circ$C, preferably 50 to 100 $^\circ$C, thereafter removing the solvent and soluble titanium compound and then heating the titanium containing support to a temperature from 80 to 200 $^\circ$C.

**Revendications**

1. Procédé de production de dimère de l'éthylène, comprenant de soumettre l'éthylène à une dimérisation en présence d'un système catalyseur de la dimérisation comprenant un catalyseur comportant un composé non halogéné du titane, qui contient trois groupes -OR ou davantage liés à l'atome de titane, où chaque R est choisi individuellement parmi les groupes hydrocarbyle contenant de 1 à 10 atomes de carbone, et un cocatalyseur comprenant un agent réducteur trialkylaluminium, **caractérisé** par l'utilisation d'un système de catalyseur où ledit catalyseur est fixé à un solide insoluble qui est un support minéral oxydé finement divisé.

2. Procédé selon la revendication 1, caractérisé en ce que ledit support est choisi parmi la silice, l'alumine, la silice-alumine, la zircone, la thorine, l'oxyde de magnésium, le phosphate d'aluminium et l'alumine phosphatée.

3. Procédé selon la revendication 2, où ledit support est le phosphate d'aluminium ou la silice.

4. Procédé selon l'une quelconque des revendications précédentes, caractérisé en ce que ledit composé de titane est choisi parmi $Ti(OC_2H_5)_4$, $Ti(OC_3H_7)_4$, $Ti(OiC_3H_7)_4$, $Ti(OnC_4H_9)_4$ et $Ti(OC_6H_4CH_3)_4$.

5. Procédé selon l'une quelconque des revendications précédentes, caractérisé en ce que ledit composé de métal de transition est le $Ti(OC_2K_5)_4$.

6. Procédé selon l'une quelconque des revendications précédentes, caractérisé en ce que ledit cocatalyseur est le triéthylaluminium.

7. Procédé selon selon l'une quelconque des revendication précédentes, caractérisé en ce que ledit support a été traité avec un agent réducteur organométallique choisi parmi les composés de la formule $MgR_2$ et $AlR'_3$, où chaque R est choisi indépendamment parmi les groupes hydrocarbyle contenant de 1 à 30 atomes de carbone et chaque R' est choisi indépendamment parmi les groupes alkyle contenant de 1 à 12 atomes de carbone.

8. Procédé selon la revendication 7, où ledit support a été traité avec l'éthyi butyl magnésium.

9. Procédé selon l'une quelconque des revendications précédentes, caractérisé en ce que ladite dimérisation est effectuée en présence, en plus, d'un catalyseur de polymérisation solide à métal de transition pour obtenir un copolymère d'éthylène.

10. Procédé selon la revendication 9, où ledit catalyseur de polymérisation solide à métal de transition contient du titane.

11. Procédé selon la revendication 1, caractérisé en ce que ledit catalyseur solide fixé est préparé en faisant réagir un cogel silice-oxyde de titane avec du méthanol pour former des liaisons entre les

9

EP 0 230 983 B1

atomes de titane et les groupes méthoxy.

12. Procédé selon la revendication 1, caractérisé en ce que ledit catalyseur solide fixé comprend un polymère de titanate de butyle comme composé à métal de transition.

13. Procédé selon la revendication 1, caractérisé en ce que ledit catalyseur a été préparé en traitant ledit support avec ledit composé de titane dans un solvant à une température de 50 à 300° C, de préférence de 50 à 100° C, puis en enlevant le solvant et le composé de titane soluble et ensuite en chauffant le support contenant le titane à une température entre 80 et 200° C

**Patentansprüche**

1. Verfahren zur Herstellung von Ethylen-Dimeren, bei dem Ethylen der Dimerisierung in Gegenwart eines Dimerisierungs-Katalysatorsystems unterworfen wird, wobei dieses System einen Katalysator unter Einschluss einer nicht-halogenierten Titanverbindung mit einem Gehalt an drei oder mehr an das Titanatom gebundenen -OR-Gruppen, wobei die einzelnen Reste R individuell unter Kohlenwasserstoffresten mit 1 bis 10 Kohlenstoffatomen ausgewählt sind, und einen Cokatalysator, der ein Trialkylaluminium-Reduktionsmittel enthält, umfasst, wobei das Verfahren dadurch gekennzeichnet ist, dass man ein Katalysatorsystem verwendet, bei dem der Katalysator an einen unlöslichen Feststoff, bei dem es sich um einen fein verteilten Träger auf der Basis eines anorganischen Oxids handelt, gebunden ist.

2. Verfahren nach Anspruch 1, dadurch gekennzeichnet, dass der feste Träger unter Siliciumdioxid, Aluminiumoxid, Siliciumdioxid-Aluminiumoxid, Zirkoniumoid, Thoriumoxid, Magnesiumoxid, Aluminium-phosphat und phosphatiertem Aluminiumoxid ausgewählt wird.

3. Verfahren nach Anspruch 2, wobei es sich beim Träger um Aluminium-phosphat oder Siliciumdioxid handelt.

4. Verfahren nach einem der vorstehenden Ansprüche, dadurch gekennzeichnet, dass die Titanverbindung unter $Ti(OC_2H_5)_4$, $Ti(OC_3H_7)_4$, $Ti(OiC_3H_7)_4$, $Ti(OnC_4H_9)_4$ und $Ti(OC_6H_4CH_3)_4$ ausgewählt wird.

5. Verfahren nach einem der vorstehenden Ansprüche, dadurch gekennzeichnet, dass es sich bei der Übergangsmetallverbindung um $Ti(OC_2H_5)_4$ handelt.

6. Verfahren nach einem der vorstehenden Ansprüche, dadurch gekennzeichnet, dass es sich beim Cokatalysator um Triethylaluminium handelt.

7. Verfahren nach einem der vorstehenden Ansprüche, dadurch gekennzeichnet, dass der Träger mit einem organometallischen Reduktionsmittel behandelt worden ist, wobei das Reduktionsmittel unter Verbindungen der Formel $MgR_2$ und $AlR'_3$ ausgewählt ist, wobei die Reste R jeweils unabhängig voneinander Hydrocarbylreste mit 1 bis 30 Kohlenstoffatomen bedeuten und die einzelnen Reste R' jeweils unabhängig voneinander Alkylreste bis 1 bis 12 Kohlenstoffatomen bedeuten.

8. Verfahren nach Anspruch 7, wobei der Träger mit Ethylbutylmagnesium behandelt worden ist.

9. Verfahren nach einem der vorstehenden Ansprüche, dadurch gekennzeichnet, dass die Dimerisierung ferner in Gegenwart eines festen Übergangsmetall-Polymerisationskatalysators unter Bildung eines Ethylen-Copolymeren durchgeführt wird.

10. Verfahren nach Anspruch 9, wobei der feste Übergangsmetall-Polymerisationskatalysator Titan enthält.

11. Verfahren nach Anspruch 1, dadurch gekennzeichnet, dass der feste, gebundene Katalysator hergestellt wird, indem man ein Siliciumdioxid-Titanoxid-Cogel mit Methanol unter Ausbildung von Bindungen zwischen den Titanatomen und den Methoxygruppen umsetzt.

12. Verfahren nach Anspruch 1, dadurch gekennzeichnet, dass der feste, gebundene Katalysator Butyltitanat-Polymer als Übergangsmetallverbindung enthält.

10

13. Verfahren nach Anspruch 1, dadurch gekennzeichnet, dass der Katalysator hergestellt worden ist, durch Behandeln des Trägers mit der Titanverbindung in einem Lösungsmittel bei einer Temperatur von 50 bis 300°C, vorzugsweise 50 bis 100°C, anschliessendes Entfernen des Lösungsmittels und der löslichen Titanverbindung und anschliessendes Erwärmen des das Titan enthaltenden Trägers auf eine Temperatur von 80 bis 200°C.